Europäisches Patentamt

European Patent Office

Office européen des brevets

(19)

(11) Publication number: **0 101 141**
**A2**

(12) **EUROPEAN PATENT APPLICATION**

(21) Application number: 83300155.5

(22) Date of filing: 12.01.83

(51) Int. Cl.³: **C 08 B 37/10**

(30) Priority: 19.07.82 US 399217

(43) Date of publication of application:
22.02.84 Bulletin 84/8

(84) Designated Contracting States:
AT BE CH DE FR GB IT LI LU NL SE

(71) Applicant: HEPAR INDUSTRIES, INC.
160 Industrial Drive
Franklin Ohio 45005(US)

(72) Inventor: Smith, Milton R.
301 Kimbary
Dayton Ohio 45459(US)

(72) Inventor: Amaya, Eduardo
811 Sands Avenue
Monroe Ohio 45050(US)

(72) Inventor: Fussi, Fernando
16 Grand Places
Fribourg(CH)

(74) Representative: Burford, Anthony Frederick et al,
W.H. Beck, Greener & Co. 7 Stone Buildings Lincoln's
Inn
London WC2A 3SZ(GB)

(54) Process for manufacturing low molecular weight heparins by depolymerization of normal heparin.

(57) Process for manufacturing low molecular weight heparin fractions from normal heparin wherein such fractions have better pharmacological and therapeutic properties than the normal heparin. The process comprises the steps of,

(a) acififying normal heparin to obtain heparinic acid with a pH in the range of about 3 to about 5, and

(b) depolymerizing the heparinic acid by heating in the presence of an oxidizing agent to obtain low molecular weight heparin fractions in the range of about 4,000 Dalton to about 12,000 Dalton.

1

# PROCESS FOR MANUFACTURING LOW MOLECULAR WEIGHT HEPARINS BY DEPOLYMERIZATION OF NORMAL HEPARIN

## BACKGROUND OF THE INVENTION

### Field of the Invention:

The present invention relates to a new and improved process for manufacturing low molecular weight heparins by the depolymerization of normal heparin. The low molecular weight heparin fractions manufactured by this process have a ratio of antithrombotic activity to anti-coagulant activity which is remarkably superior to that of the normal heparin.

### The Prior Art:

One method of manufacturing low molecular weight heparins having superior pharmacological and therapeutic properties is disclosed in Dr. Fussi's United States Patent No. 4,281,108. In that patent, low molecular weight heparins having a ratio of antithrombotic activity to anticoagulant, i.e., anticlotting, activity superior to that of normal heparin is produced by a process comprising the following steps,

(a) acidification of normal heparin to obtain heparinic acid,

(b) depolymerization of said heparinic acid by heating in the presence of peroxides to obtain a low molecular weight heparamine, and

(c) sulphation of said heparamine to obtain the low molecular weight heparin.

The heparin fractions produced in accordance with the disclosed process does have a ratio of antithrombotic activity to anticoagulant activity significantly greater than 1 to 1 as compared to that of normal heparin which for the present purpose has a ratio of 1:1. Although, the disclosed process was successful in producing this superior low molecular weight heparin, the depolymerization step caused the detaching of some N-sulfate groups which necessitated the sulphation (step C) in order to reintroduce the lost N-sulfate groups. Moreover, some impurities are also introduced into the final product during the sulphation.

## OBJECT OF THE PRESENT INVENTION

The object of the present invention is to develop a process wherein normal heparin can be depolymerized but without detaching N-sulfate groups while still producing low molecular weight heparin having the aforesaid superior ratio of antithrombotic to anticoagulant activity.

Surprisingly, this has been accomplished by conducting the depolymerization in the presence of a peroxide at a pH range of about 3 to about 5. In this pH range, the depolymerization does not affect the sulfate groups and unexpectedly gives a better yield of depolymerized fraction, eliminates the sulphation step, and also produces a purer product.

## DESCRIPTION OF THE INVENTION

Heparin is a well-known and stable uni-or bivalent salt of the unstable heparinic acid. It is a polymer of a disaccharide unit formed by hexuronic (D-glucuronic and L-iduronic) acids and glucosamine, O- and N- sulphates, linked by alpha 1-4 glycosidic linkages.

Heparin is present in different forms in many tissues and cells and is, more or less, loosely bound to a protein moiety. In the skin and, partially, in the lungs of different species, heparin is present in a high molecular form. In this form, heparin is sensitive to the action of ascorbic acid, or of various enzymes believed present in intestinal mucosa. After treatment with either ascorbic acid or intestinal mucosa homogenates, these macromolecular forms of skin or lung heparin can be reduced to the same molecular size as heparin isolated from intestinal mucosa.

Heparin isolated from intestinal mucosa having a mean molecular weight ($\overline{MW}$) of 15,000 Dalton, or heparin from other sources such as skin or lungs which has been treated, as discussed above, with either ascorbic acid or intestinal mucosa homogenates, have the smallest possible molecular size for natural heparin, i.e. about 15,000 Dalton as a mean molecular weight. For purposes of the present invention such heparin will be referred to as normal heparin.

In fact, there are no enzymes in the body which can split heparin obtained from intestinal mucosa into lower molecular weight fractions, nor have any chemical methods other than that disclosed in United States Patent No. 4,281,108 been discovered for depolymerizing normal heparin without a total loss of biological activity. However, as discussed above, that process has its disadvantages.

Heparin was first discovered and isolated from tissues in 1917 and from that date has been known for its anticoagulant (anticlotting) ability. Recently, the anticoagulant ability, i.e., activity, has been evaluated in conjunction with its antithrombotic activity. For pharmacological and therapeutic purposes, the modified ratio either in vitro in vivo of antithrombotic activity to anticlotting (anticoagulant) activity is the most important factor. This is measured by the following ratio:

$$\frac{\text{Anti Xa test}}{\text{APTT}}$$

where: Anti Xa test = Yins' test

APTT = Activated partial thromboplastin time Normal heparin, i.e., $\overline{MW}$ of about 15,000 Dalton has a total anticlotting activity $\cong$ 150 IU/mg and a $\frac{\text{anti Xa test}}{\text{APTT}}$ = 1, while heparins produced in accordance with the process of United States Patent No. 4,281,108 and the present process have a total anticlotting activity $<$ 150 IU/mg and a $\frac{\text{anti Xa test}}{\text{APTT}}$ $>$ 1.

Interestingly, compound IV of United States Patent No. 4,281,108 has a ratio of almost 2 which means that such heparin has an antithrombotic activity twice that of normal heparin based on the same anticlotting activity; in other words, in terms of anticlotting activity, half a dose of such compound is required to produce the same antithrombotic action as is produced by a whole dose of normal heparin.

Surprisingly, the present invention results in a low molecular weight heparin with the same advantageous ratio but without the need for the sulphation step. Also, surprisingly, the yields of the process of the present invention are superior to that obtained by United States Patent No. 4,281,108 and the resultant product is purer which eliminates the need for further purification.

The process of the present invention comprises the following steps,

(a) acidifying normal heparin to obtain heparinic acid with a pH in the range of about 3 to about 5, and

(b) depolymerizing the heparinic acid in the presence of peroxides to form low molecular weight fractions of heparin.

The heparin, so produced, has a $\overline{MW}$ ranging between about 4,000 and 12,000 Dalton and has the same superior ratio as that of low molecular weight heparin functions produced in accordance with the process of United States Patent No. 4,281,108. In addition, the yield of such low molecular weight fractions is improved as well as producing a purer product.

This process also has the advantage of producing low molecular weight heparin fractions ranging from about 4,000 to 12,000 Dalton. Thus, it is possible to choose fractions with differing ratios of antithrombotic activity to anticlotting activity for differing therapeutic and pharmacological purposes.

This offers many advantages. For example, a patient in thrombogenic condition, with very shortened clotting time, might use an antithrombotic agent with a fairly high anticlotting activity. On the other hand, a patient being submitted to a surgical intervention having both the risk of hemorrhage and postoperative thrombosis, might use an antithrombotic agent with a low anticlotting activity.

The acidification step of the present process can be accomplished by the simple acidification of normal heparin with known acids, or preferably, by treatment with cation exchange resin. The important factor in this step is producing heparinic acid with a pH in the range of about 3 to about 5.

The heparinic acid is then depolymerized in the presence of an oxidizing agent. The depolymerization may be conducted, for example, in an autoclave in the presence of hydrogen peroxide. In such a case, the autoclave is heated at a pressure preferably between about 1 and about 2 atmospheres. The autoclaving can be stopped at preselected intervals such as at 15, 30, 60, 120 and 240 minutes to obtain low molecular weight heparin fractions with varying $\overline{MW}$. There $\overline{MW}$ will vary from about 4,000 to 12,000 $\overline{MW}$ Dalton depending upon the time of autoclaving with the longer periods producing the smaller fractions.

While the process of United States Patent No. 4,281,108 yields about 65% in polymerized fractions, the yields of the present process are better. In addition, a purer final product is produced which is free of foreign substances such as free pyridine, trimethyl amine and unreacted sulfotrioxides which are introduced into the product of United States Patent No. 4,281,108 as a result of the requisite sulphation step. Thus, the need for any purification step is eliminated.

The process of the present invention will be further illustrated by the following example which in no way is to be considered limiting.

EXAMPLE

70 grams of commercial heparin is dissolved in 350 ml of distilled water and cooled to 5-10°C. A suitable strong cationic resin is added until the pH drops to 3.0-3.5 and the resin is removed by filtration. 14 ml of hydrogen peroxide is added and the solution immediately autoclaved for 10 minutes at 125°C. and 21 psi. After the solution is cooled, the pH is adjusted to 6.8 with sodium hydroxide and ethyl alcohol is added until a concentration to about 50% is reached. The precipitate is collected and dried under vacuum.

## WHAT IS CLAIMED IS:

1.  Process for obtaining low molecular weight heparin fractions having elevated pharmacological properties comprising the steps of,

     (a)  acidifying normal heparin to obtain heparinic acid with a pH in the range of from about 3 to about 5, and

     (b)  depolymerizing said heparinic acid by heating in the presence of an oxidizing agent to obtain low molecular weight heparin fractions in the range of about 4,000 Dalton to about 12,000 Dalton.

2.  Process of claim 1 wherein said pH of step (a) is in the range of about 3.0 to about 3.5.

3.  Process of claim 1 wherein said depolymerizing takes place in an autoclave.

4.  Process of claim 1 wherein said acidifying is with a cation exchange resin.

5.  Process of claim 1, wherein said oxidizing agent is selected from at least one of hydrogen peroxide.